# EUROPEAN PATENT APPLICATION

(11) **EP 4 253 399 A1**
(43) Date of publication of application: **04.10.2023**
(21) Application number: 21898766.7
(22) Date of filing: 19.11.2021
(51) Int. Cl.: C07K 7/00, A61K 38/10, A61K 39/395

(54) **SCAFFOLDING PROTEIN FOR GENERATING ANTIGEN-BINDING CHIMERAL PROTEINS**

(30) Priority: 27.11.2020 MX 2020012914
(71) Applicant: Centro de Investigación Científica y de Educación Superior de Ensenada, Baja California (CICESE), 22860 Ensenada, Baja California (MX)
(72) Inventor: LICEA NAVARRO, Alexei Fedorovish, 22860 Baja California (MX); SÁNCHEZ CAMPOS, Liliana Noemí, 22860 Baja California (MX); DUEÑAS ESPINOZA, Salvador, 22860 Baja California (MX)
(74) Representative: Urizar Anasagasti, Jesus Maria
(86) International application number: PCT/MX2021/050077
(87) International publication number: WO 2022/114946

(57) **Abstract**

Disclosed is a scaffolding protein having SEQ ID NO: 1, derived from a conotoxin, which does not have amino acid residues associated With the toxic activity of the conotoxin and Which can be used to generate chimeral proteins having 19-47 amino acids, fragments of a CDR3 of a vNAR With affinity for a specific antigen being inserted between amino acids 11 and 12 thereof.

## Description

### FIELD OF INVENTION

The present invention relates to a small-sized scaffolding protein that can be used to generate chimeric proteins, and more specifically, it relates to a novel conotoxin-derived scaffold sequence, which is useful for generating antigen-binding chimeric proteins by fusing the scaffold with a CDR3 from a vNAR domain, and that retained recognition of the antigen to which the vNAR was directed.

### BACKGROUND OF THE INVENTION

The use of antigen-binding proteins as signaling molecules or for blocking metabolic pathways has significantly improved the detection and treatment of various pathologies associated with the overexpression of metabolites, or the presence of altered metabolic pathways. Unlike most commercial drugs or signaling molecules, the use of antigen-binding proteins ensures greater specificity of treatment or detection, since the proteins have a very high affinity for the binding site with the target antigen, therefore, when used, in most cases, the presence of false positives in detection or non-specificity in treatment is avoided.

However, the use of antigen-binding proteins is not without problems, since most of the available antigen-binding proteins are thermolabile, which makes their storage and transport difficult, so that their use as agents of labeling, or treatment, is limited by the problems associated with the preparation of effective formulations, which maintain the stability of the protein without affecting its functionality. The vast majority of detection kits, or drugs that include antigen-binding proteins as the active molecule, must be stored under very strict temperature conditions and quickly lose their stability when moving from storage to room temperature.

Another of the limitations of the large size of conventional antigen-binding proteins is that it is difficult for them to cross the biological barriers of multicellular organisms, so their effect as a treatment or means of detection is limited to the superficial layers of the tissue where they are applied. Therefore, most of the current treatments require multiple applications of the antigen-binding protein, as well as alternate application mechanisms that improve the rate of penetration into the tissue in order to carry out its effect.

In order to improve the use of antigen-binding proteins as markers or therapeutic agents, attempts have been made to carry out various types of modifications to them, either to improve their penetration rate, or to increase their thermal stability against to sudden changes in temperature; however, most of the modifications made tend to decrease the binding strength of the protein with the antigen binding site to which it is directed and, in many cases, the functionality of the protein is completely lost.

In view of the above problems, there is a need to provide a scaffolding protein of reduced size, which can be used as a framework for the generation of chimeric proteins that retain affinity for the binding site, and which also allows the use of small size fragments of a CDR3 from a vNAR with specific affinity against antigen, in order to obtain a chimeric protein of small size, so that a higher rate of penetration into the tissues is present.

### SUMMARY OF THE INVENTION

In order to overcome the drawbacks of available antigen-binding proteins, the present invention aims to provide a conotoxin-derived scaffolding protein capable of serving as a framework for the generation of antigen-binding chimeric proteins.

A particular objective of the present invention is to provide a scaffolding protein that, when inserted into it a CDR3 sequence from a parental vNAR, preserves the affinity for the antigen presented by the parental vNAR.

Another objective of the present invention is to provide a scaffolding protein that makes it possible to obtain chimeric proteins of small size, preferably between 19 and 47 amino acids.

An additional objective of the present invention is to provide a scaffolding protein that, when inserted into it a CDR3 sequence from a vNAR, presents a greater capacity for penetration into tissues and greater thermal stability.

The foregoing, as well as other, objects and advantages of the present invention will become apparent from the following detailed description thereof.

### DESCRIPTION OF THE FIGURES OF THE INVENTION

Figure 1 illustrates a graph showing the results of the evaluation of the activity of the cal 14.1a scaffold, and how it loses its toxin activity when adding the CDR3 of a vNAR.
Figure 2 shows a graph with the results of the recognition ELISA assay, where the interaction of the cal_P98Y chimeric protein with VEGF₁₆₅ is shown.
Figure 3 shows a series of images in which the behavior of the HUVEC cell line is observed in *in-vitro* neutralization assays with cal_P98Y.
Figure 4 shows a graph summarizing the results of the in-vitro neutralization assay with cal_P98Y.

### DETAILED DESCRIPTION OF THE INVENTION

The present invention provides a scaffolding protein having the following amino acid sequence; 
SEQ ID NO: 1

Gly-Asp-Cys-Pro-Pro-Trp-Cys-Gly-Ala-Arg-Cys-(XXX)-Cys

1 2 3 4 5 6 7 8 9 10 11 12
 where (XXX) represents the scaffolding protein insertion site.

The SEQ ID NO:1 scaffolding protein of the present invention can be used as a framework for the rational design of antigen-binding chimeric proteins, with a final size between 19 and 47 amino acids. To obtain the aforementioned chimeric protein, a fragment of a CDR3 of a parental vNAR with affinity for a specific antigen, with a length of between 7 and 35 amino acids between positions 11 and 12, is inserted into said scaffolding protein, in such a way that, a chimeric protein is obtained that retains the affinity for the antigen to which the CDR3 of the parental vNAR was directed. The generated chimeric protein is subjected to an *in silico* analysis process to determine its theoretical stability, as well as its antigen binding strength. The selection process consists of performing the homology modeling of the chimeric protein with the MODELLER program. Subsequently, the refinement is carried out by means of molecular dynamics simulations with the NAMD program. Once the structures have been obtained, the protein-protein interaction analysis (Docking) is carried out with the ClusPro program, evaluating different binding and orientation zones in order to find the optimal structural conformation through two stages of docking. The first step consists of determining the contact patterns and the second step is based on the energetic analysis of the contact regions of each of the proteins in the ROSIE platform, using as a positive control, the interaction of the CDR3 of the parental vNAR with the antigenic molecule, to obtain a reference binding energy. In this way, the chimeric proteins generated, which present a binding strength of between 85 and 150%, with respect to the reference binding strength, are selected to be used.

The above-described construction allows the formed chimeric protein to maintain the ability to bind to the same epitope of the parent antibody, without losing effectiveness. In addition, due to the smaller size of the chimeric protein obtained, compared to the size of the parental antibody, it has a greater capacity to penetrate tissues. Likewise, due to their conformation, the chimeric proteins obtained present an improvement in their thermal stability, which increases their resistance to temperature changes.

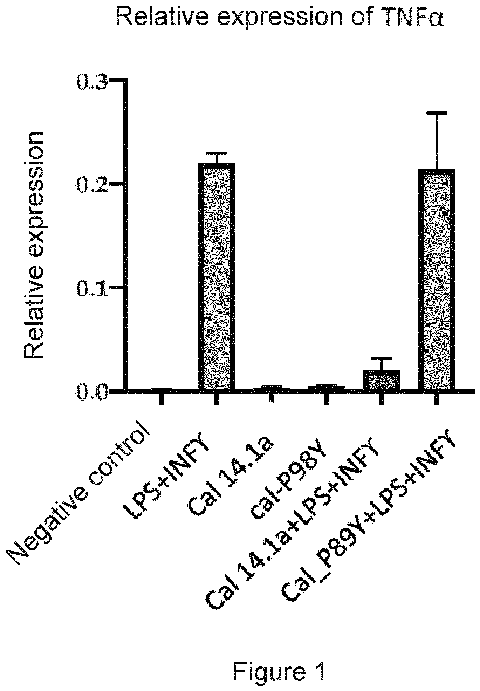

The SEQ ID NO:1 scaffolding protein of the present invention, even when it is based on the structure of a conotoxin, does not present any type of toxic activity, since it does not present any amino acid sequence associated with the activity of the toxin (Figure 1), so it can be used as a medicine in pathologies associated with the expression of a molecule or the blocking of a metabolic pathway. Likewise, the chimeric proteins obtained can be used in *in vitro* detection processes of antigenic molecules, such as, for example, those produced by the presence of a pathology.

The chimeric proteins obtained by inserting a fragment of a CDR3 of a vNAR with affinity for a specific antigen between amino acids 11 and 12 of SEQ ID NO: 1, have a size much smaller than that of an IgG type antibody, therefore, they are characterized by having greater penetration into tissues and also present two disulfide bonds in the scaffolding protein, which considerably improve their thermal stability, so that when used as an active component of a drug, they allow obtaining a better rate of penetration into tissues and also significantly improve their thermal stability, which has an impact on better handling and storage of the drug in which the chimeric proteins obtained with the present invention are used.

The chimeric proteins obtained using the scaffolding protein of SEQ ID NO: 1 of the present invention can be used in the manufacture of medicines intended to treat pathologies associated with the expression of a particular antigenic molecule produced by autoimmune diseases, infectious diseases, chronic-degenerative diseases and neoplasms. For example, chimeric proteins using the scaffolding protein of SEQ ID NO: 1 of the present invention can be designed to interact with antigenic molecules produced in various pathologies.

In addition, the chimeric proteins obtained using the scaffolding protein of SEQ ID NO:1 of the present invention can alternatively be used as antigen detection molecules *in vitro.* Due to their high thermal stability, the chimeric proteins obtained according to the teachings of the present invention can be placed in detection kits, being stored for longer periods than would be possible with other types of proteins.

In order to make more evident the technical advantages obtained by using the scaffolding protein of SEQ ID NO: 1 of the present invention, several chimeric proteins directed against different unrelated antigenic targets were generated, as shown in Table 1.

**Table 1. Parental antibodies used and chimeric proteins generated.**

| Target antigen | CDR3 vNAR | obtained chimeric protein |
|---|---|---|
| VEGF₁₆₅ | C-P98Y | GDCPPWCGARCKNLLPRYLVNGIAAMGYSSSC |
| TGF-β | N-T 10 | GDCPPWCGAPCHTKWGFFPLSWKLVGAALINRSC |
| TGF-β | N-T21 | GDCPPWCGAPCQAKKWWAADPLLFLIGRWLNVGC |
| CEA | N-CV043 | GDCPPWCGARCDMVWSWWGGWRPVRRLGWKGWSC |
| TNF-α | C-T16 | GDCPPWCGARCKAQGLIDTSVRGLAVPGNCERCSSYHC |
| TNF-α | C-T65 | GDCPPWCGARCKARESDYNRVGIRDYKDC |
| PCSK9 | N-PK13 | GDCPPWCGARCARVWVSWVARAFFRGINFLPVFSC |
| AMA1 | N-14I-1 | GDCPPWGARCFYSLPLRDYNYSLLC |
| Lysozyme | N-A07 | GDCPPWCGARCESRYGSYDAECAALNDC |
| SPIKE SARS-CoV-2 | N-MSP24 | GDCPPWCGARCRSVLQVGVSVGGLAYYC |

Once the chimeric proteins were generated from the binding of the selected fragment of the chosen parental antibody with the scaffolding protein of SEQ ID NO:1, the *in silico* binding strength thereof, with the binding site was compared with respect to the binding strength with the binding site of its parent antibody. Likewise, its thermal stability was evaluated in a comparative way. A summary of the results obtained is shown in Table 2.

**Table 2. Comparison between the binding strength and the thermal stability of the parental antibodies against their chimeric proteins.**

| | | Binding strength | Organism |
|---|---|---|---|
| VEGF₁₆₅ | Cal_P98Y | -41.54 REU | *Heterodontus fransisci* |
| | P98Y | -45.54 REU | |
| VEGF₁₆₅ | Cal_VS04 | -47.07 REU | *Heterodontus fransisci* |
| | VS04 | -49.00 REU | |
| TGF-β | Cal_ T10 | -27.24 REU | *Heterodontus fransisci* |
| | T10 | -34.55 REU | |
| TGF-β | Cal_T21 | -34.02 REU | *Heterodontus fransisci* |
| | T21 | -38.26 REU | |
| TNF-α | Cal_T16 | -27.05 REU | *Heterodontus fransisci* |
| | Tn16 | -33.01 REU | |
| TNF-α | Cal_T65 | -28.34 REU | *Heterodontus fransisci* |
| | Tn65 | -34.01 REU | |
| PCSK9 | Cal_pk13 | -28.20 REU | *Heterodontus fransisci* |
| | PK13 | -20.39 REU | |
| AMA1 | Cal_AMA1 | -37.18 REU | *Orectolobus maculatus* |
| | 14I-1 | -36.53 REU | |
| Lysozyme | Cal_lis | -29.78 REU | *Ginglymostoma cirratum* |
| | A07 | -32.29 REU | |
| SPIKE SARS-CoV-2 | Cal_MSP24 | -25.37 REU | *Heterodontus fransisci* |
| | MSP24 | -34.18 REU | |

As can be seen, some of the chimeric proteins generated, collectively presented a greater binding strength with the binding site than their parental antibodies and, in addition, this strategy was tested with three sharks that are currently used to generate vNAR for commercial purposes, so it is clear that the described invention can be carried out in any suitable species of shark.

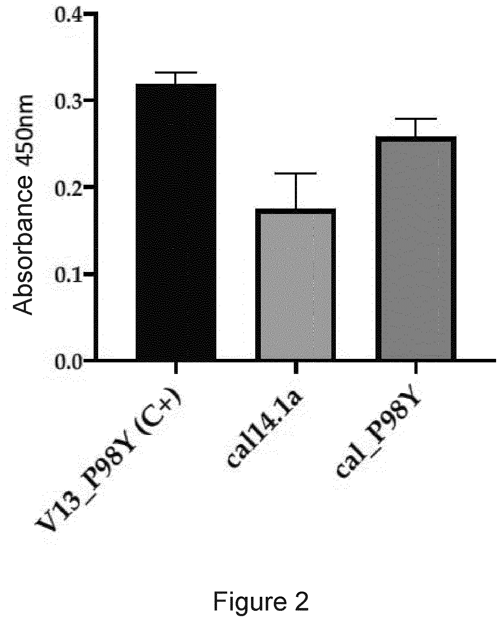

To demonstrate the function of the chimeric proteins obtainable in accordance with the teachings of the present invention, in-vitro assay of cal 14.1a scaffold activity was performed. The "complete" scaffold has the effect of inhibiting TNF expression in HUVEC cell culture. As can be seen in figure 1, the negative control that did not express TNF in the culture is observed. When LPS+IFN was added, TNF expression increased, when only the scaffold was added, there was no expression, and when the chimeric was added, there was no expression. These last two trials were without LPS or Interferon. On the other hand, the "complete" scaffold is observed plus LPS and Interferon, and it is seen how the scaffold inhibited the TNF expression (toxin effector function). However, when the chimeric protein plus LPS and Interferon were added, no inhibition of TNF expression was observed. Therefore, the chimeric protein did not inhibit, that is, the scaffold loses its biological activity and only works as a scaffold to place other sequences.In another test, an ELISA assay was performed to identify the interaction of cal_P98Y chimeric protein with VEGF₁₆₅ (Figure 2). For this, the comparison with its parental vNAR was made. As can be seen in the graph shown in Figure 2, the chimeric protein has the ability to recognize the antigen against which the parental vNAR is targeting.

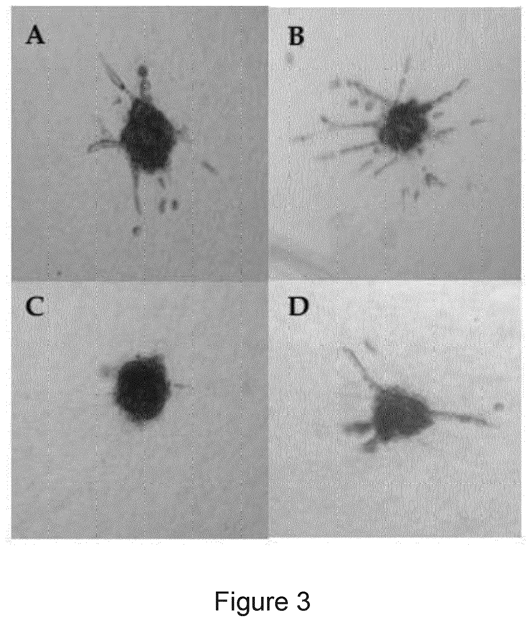

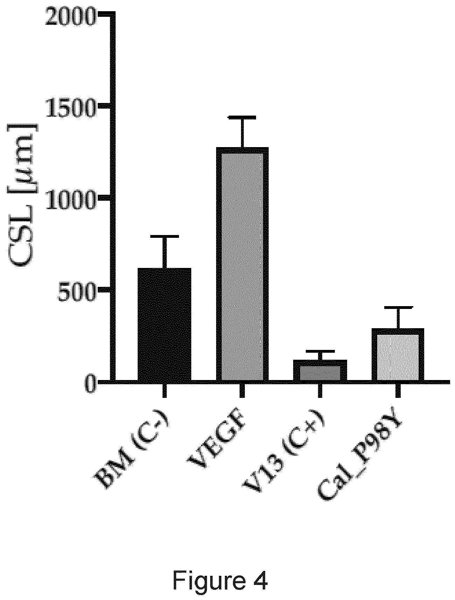

Finally, an *in vitro* neutralization assay was performed. As can be seen in figure 3, VEGF generates angiogenesis. In panel A, HUVEC cells are observed in culture medium, with little angiogenesis; in panel B, cells plus VEGF are observed, therefore angiogenesis is increased; in panel C, cells plus VEGF plus the parental antibody are seen, observing an inhibition of angiogenesis and; in panel D, cells plus VEGF plus the chimeric molecule are seen, and the inhibition of angiogenesis is appreciated with respect to panel B. Figure 4 shows the graphical representation of the four experimental conditions shown in the panels of the Figure 3, showing that the Cal_P98Y chimeric protein inhibits VEGF, which corroborates that the present invention makes it possible to obtain functional chimeric proteins.

The present invention has been described in accordance with one of its preferred embodiments; however, it will be apparent to a technician with average knowledge in the matter, that modifications may be made to the invention, without departing from its spirit and scope.

## Claims

1. A scaffolding protein derived from a conotoxin of SEQ ID NO: 1: 
Gly-Asp-Cys-Pro-Pro-Trp-Cys-Gly-Ala-Arg-Cys-(XXX)-Cys
1 2 3 4 5 6 7 8 9 10 11 12
 where (XXX) represents the scaffolding protein insertion site.

2. An antigen-binding chimeric protein **characterized in that** it comprises the scaffolding protein of claim 1 and; a fragment between 7 and 35 amino acids of a CDR3 of a vNAR with affinity for a specific antigen, which is inserted at the insertion site (XXX) between amino acids 11 and 12 of SEQ ID NO: 1, provided that said CDR3 fragment from vNAR generates a chimeric protein exhibiting an antigen binding strength of between 85 and 150% relative to the antigen binding strength of CDR3 from vNAR.

3. The antigen-binding chimeric protein according to claim 2, **characterized in that** the inserted fragment is a CDR3 fragment from a shark vNAR.

4. The antigen-binding chimeric protein according to claim 3, **characterized in that** the CDR3 fragment from shark vNAR is selected from the group consisting of CDR3 fragments of vNAR from *Heterodontus fransisci, Orectolobus maculatus and Ginglymostoma cirratum* sharks.

5. The use of the scaffolding protein of claim 1, for the generation of antigen-binding chimeric proteins, wherein is inserted between amino acids 11 and 12, into said scaffolding protein, a fragment of between 7 to 35 amino acids of a CDR3 from a vNAR with affinity for a specific antigen.

6. The use of the chimeric protein according to claim 2, as a medicine.

7. A pharmaceutical composition comprising a scaffolding protein according to claim 1 or a chimeric protein according to claim 2 and; a pharmaceutically acceptable vehicle.

8. The pharmaceutical composition according to claim 7, for the treatment of a pathology associated with the expression of an antigenic molecule.

9. The pharmaceutical composition according to claim 7, **characterized in that** it is adapted to be administered systemically or locally.

10. The pharmaceutical composition according to claim 8, **characterized in that** the pathology associated with the expression of an antigenic molecule is selected from the group consisting of autoimmune diseases, infectious diseases, chronic-degenerative diseases and neoplasms.

11. The use of the scaffolding protein of claim 1 or the chimeric protein of claim 2 to treat a pathology associated with the expression of an antigenic molecule.

12. The use according to claim 11, wherein the scaffolding protein and the chimeric protein are adapted to be administered systemically or locally.

13. The use according to claim 11, wherein the pathology associated with the expression of an antigenic molecule is selected from the group consisting of autoimmune diseases, infectious diseases, chronic-degenerative diseases, and neoplasms.

14. The use of the scaffolding protein of claim 1 or the chimeric protein of claim 2, in a kit for the *in vitro* detection of antigenic molecules.

15. The use of the pharmaceutical composition according to claim 7, for the treatment of a pathology associated with the expression of an antigenic molecule.

16. The use according to claim 15, wherein the pathology associated with the expression of an antigenic molecule is selected from the group consisting of autoimmune diseases, infectious diseases, chronic-degenerative diseases, and neoplasms.

17. The use according to claim 15, wherein the composition is adapted to be administered systemically or locally.

18. The use of the chimeric protein of claim 2, to detect or quantify antigens.

19. The use of the chimeric protein of claim 2, in the manufacture of detection tests for mammalian pathogens.
